# EUROPEAN PATENT APPLICATION

(11) **EP 2 522 318 A1**
(43) Date of publication of application: **14.11.2012**
(21) Application number: 11165884.5
(22) Date of filing: 12.05.2011
(51) Int. Cl.: A61F 9/00

(54) **Guide device for intraocular injection**

(71) Applicant: Sanofi-Aventis Deutschland GmbH, 65929 Frankfurt (DE)
(72) Inventor: The designation of the inventor has not yet been filed
(74) Representative: KEIL & SCHAAFHAUSEN Patentanwälte

(57) **Abstract**

Described is a guide device (10) for intraocular injection comprising a base plate (11) having an inner surface (13) and an outer surface (19), a handle (17) formed on the outer surface (19), and a hole (15) formed in the base plate (11) and extending through the inner and outer surfaces. The inner surface (13) is adapted to contact an eye ball.

## Description

### Background

The present invention relates to a guide device for intraocular injection and a system comprising this guide device and an injection device.

A number of vision-threatening disorders or diseases of the eye need to deliver a medicament (e.g., pharmaceutical, proteins, antibodies, implantable devices, etc.) to a posterior segment of the eye by intraocular delivery (more specifically intravitreal delivery). One such technique for intraocular delivery is accomplished by intraocular injection directly into the vitreous body.

Conventionally, the eye lids are clamped open, and a physician measures an appropriate distance from the cornea of the eye to identify the injection site. Then, a needle is inserted into the injection site and injects the medicament. After that, the needle is removed, and the injection site is closed with a pliers type tool, until the medicament is believed to have dissipated. By closing the injection site, leakage of the medicament out of the injection site is minimized. Document US 2010/0100054 A1 discusses an injection system for intraocular injection.

The conventional method of administered the intraocular injection requires several devices and post-injection tools for properly administering the injection. Hence, it is an object of the present invention to provide a device for facilitating an intraocular injection.

### Summary

In an exemplary embodiment, a guide device for intraocular injection comprises a base plate having an inner surface and an outer surface, a handle formed on the outer surface, and a hole formed in the base plate and extending through the inner and outer surfaces. The inner surface is adapted to contact an eye ball. The inner surface may comprise a first convex portion adapted to cover a cornea of the eye ball and a second convex portion surrounding a periphery of the first portion and adapted to cover a portion of the eye ball surrounding the cornea.

In an exemplary embodiment, a first radius of curvature of the first convex portion is greater than a second radius of curvature of the second convex portion. The hole may be located at a predetermined distance from the periphery of the first portion. The handle may be an elongated rib extending substantially across a width of the base plate.

In an exemplary embodiment, the base plate comprises first opposing edges to abut respective eyelids of the eye ball. The first opposing edges may include barriers formed on the outer surface to abut the respective eyelids.

In an exemplary embodiment, the inner surface includes a medicament which may be at least one of an antimicrobial agent, an anti-infection agent, and an anti-bacterial agent.

In an exemplary embodiment, the base plate is rotatable relative to the eye ball between at least a first orientation and a second orientation.

These as well as other advantages of various aspects of the present invention will become apparent to those of ordinary skilled in the art by reading the following description, with appropriate reference to the accompanying drawings.

### Brief Description of the Drawings

Exemplary embodiments are described herein with reference to the schematic drawings in which:
- Fig. 1: illustrates an exemplary embodiment of a guide device for intraocular injection in a back view according to the present invention;
- Fig. 2: shows the embodiment of Fig. 1 in a first side view;
- Fig. 3: shows the embodiment of Fig. 1 in a second side view; and
- Figures 4 to 8: illustrates an exemplary embodiment of a sequence for using the guide device of Figures 1 to 3 with an eye according to the present invention.

### Detailed Description

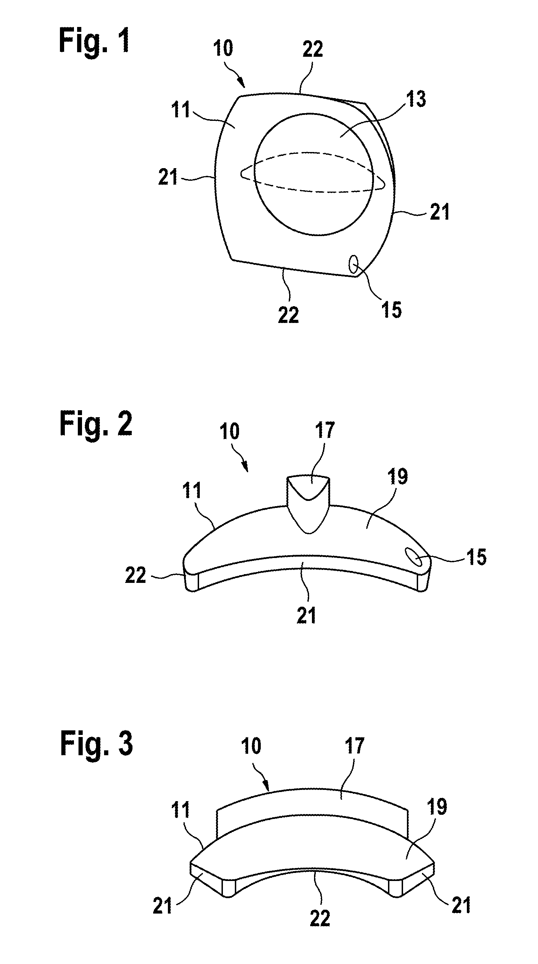

Figure 1 shows an exemplary embodiment of a guide device 10 for intraocular injection according to the present invention. In an exemplary embodiment, the guide device 10 comprises a base plate 11 having two pairs of opposing edges, namely first opposing edges 21 and second opposing edges 22, and a convex form, such that the base plate 11 easily adapts to the outer surface of an eye ball 32. Those of skill in the art will understand that the base plate 11 may be square, rectangular, circular, ellipsoidal, star-shaped, or a variety of other shapes.

In an exemplary embodiment, the base plate 11 includes a contoured inner surface 13 such that the guide device 10 may center itself over the cornea of the eye ball 32. For example, the inner surface 13 may include a first portion having a first convex shape (e.g., similar to that of a contact lens) and a second portion surrounding a periphery of the first portion and having a second convex shape to match that of an exposed portion of the eye ball 32 surrounding the cornea. When the guide device 10 is placed on the eye ball 32, it may be positioned such that the cornea fits at least partially within the first portion. Abutment of the cornea to the first portion may prevent the guide device 10 from moving relative to the eye ball 32 during and after an injection procedure. In an exemplary embodiment, a radius of curvature of the first portion may be greater than a radius of curvature of the second portion, such that the first portion accommodates the cornea and the second portion accommodates the eye ball 32.

In an exemplary embodiment, the base plate 11 further comprises a through hole 15 for aligning a needle of an injection device. The hole 15 may be formed at a predetermined distance from the periphery of the first portion. The predetermined distance may be determined as a function of a desired injection site. For example, to ensure that the injection site is not aligning with the cornea or the lens (unless that is the desired target), the hole 15 may be formed on a periphery of the base plate 11. A diameter of the hole 15 may be substantially equal to (or slightly greater than) a diameter of a needle used to administer the injection.

In an exemplary embodiment as shown in Figure 2, a handle 17 is formed on an outer surface 19 of the base plate 11. The handle 17 may allow a physician to rotate the guide device 10 relative to the eye ball 32. In the exemplary embodiment shown in Figure 2, the handle 17 is formed as an elongated rib extending across a width of the base plate 11. However, those of skill in the art will understand that the handle 17 may be other shapes or sizes, and a height of the handle 17 may be optimized for gripping by hand or by a medical instrument (e.g., forceps). Other forms are possible as well, for example a knob or a handle.

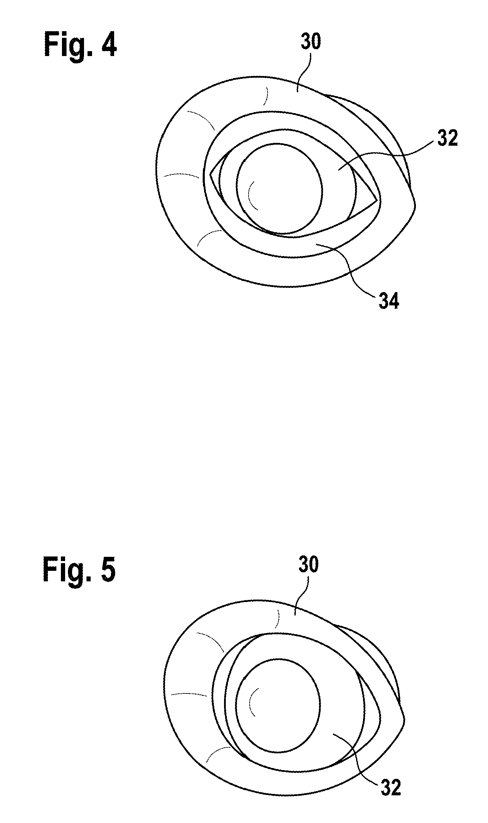

Figures 4 to 8 show an exemplary embodiment of a method of using the guide device 10 according to the present invention.

As shown in Figures 4 and 5, an eye 30 is shown in an initial position. At first, eye lids 34 of the eye 30 are separated, and the eye ball 32 may be cleaned or otherwise prepared for an injection.

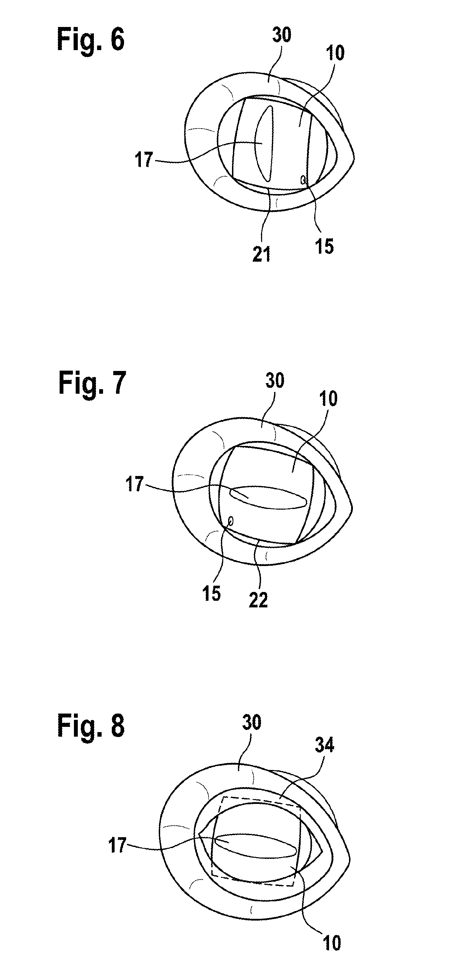

As shown in Figure 6, the guide device 10 is placed in a first orientation in contact with the eye ball 32. In an exemplary embodiment, in the first orientation, the handle 17 may be parallel to a sagittal plane, such that terminal ends of the handle 17 abut the respective eye lids to maintain separation of the eye lids during the injection procedure.

In another exemplary embodiment, the first and/or second opposing edges 21, 22 may be contoured in accordance with a shape of the eye. For example, one pair of the first and second opposing edges 21, 22 may have opposing concave and convex contours to abut opposing eye lids. In this manner, the guide device 10 may be utilized to maintain separation of the eye lids during the injection. In an exemplary embodiment, barriers may be formed on the first and/or second opposing edges 21, 22 to abut the eye lids and provided positional stability to the guide device 10. In the first orientation, the hole 15 may be aligned with the desired injection site, and the needle may pierce the eye ball 32 through the hole 15.

As shown in Figure 7, after the injection has been delivered and the needle has been withdrawn from the eye ball 32, the guide device 10 may be rotated into a second orientation using the handle 17 such that the injection site is covered by the base plate 11. In an exemplary embodiment, the guide device 10 may be rotated by approximately 90° relative to the eye ball 32. After rotating the device 10, the handle 17 may be parallel to a transverse plane, allowing the eye lids 34 to close over the guide device 10, as shown in Figure 8. Allowing the eye lids 34 to close after the procedure may be more comfortable for the patient.

After the guide device 10 has been placed in the second orientation, the injection site is covered by the base plate 11 (because the hole 15 is offset from the injection site). Thus, covering the injection site may prevent the medicament from leaking out of the eye ball 32.

Covering the injection site may also promote faster healing and prevent infection. For example, at least a portion (e.g., the second portion or part thereof) may be coated with a medicament (e.g., an antimicrobial agent, an anti-infection agent, an anti-bacterial agent, etc.) to promote healing of the injection site and/or prevent infection.

Those of skill in the art will understand the modifications (additions and/or removals) of various components of the device and/or system and embodiment described herein may be made without departing from the full scope and spirit of the present invention, which encompass such modifications and any and all equivalents thereof.

### Reference signs

- 10: guide device
- 11: base plate
- 13: inner surface of the base plate 11
- 14: through hole
- 17: handle
- 19: outer surface of the base plate 11
- 21: first edge
- 22: second edge
- 30: eye
- 32: eye ball
- 34: eye lid

## Claims

1. A guide device (10) for intraocular injection, comprising:
a base plate (11) having an inner surface (13) and an outer surface (19), wherein the inner surface (13) is adapted to contact an eye ball;
a handle (17) formed on the outer surface (19); and
a hole (15) formed in the base plate (11) and extending through the inner and outer surfaces.

2. The guide device (10) according to claim 1, wherein the inner surface (13) comprises:
a first convex portion adapted to cover a cornea of the eye ball; and
a second convex portion surrounding a periphery of the first portion and adapted to cover a portion of the eye ball surrounding the cornea.

3. The guide device (10) according to claim 2, wherein a first radius of curvature of the first convex portion is greater than a second radius of curvature of the second convex portion.

4. The guide device (10) according to claim 2, wherein the hole (15) is located at a predetermined distance from the periphery of the first portion.

5. The guide device (10) according to any one of the preceding claims, wherein the handle (17) is an elongated rib extending substantially across a width of the base plate (11).

6. The guide device (10) according to any one of the preceding claims, wherein the base plate (11) comprises:
first opposing edges (21) to abut respective eyelids of the eye ball.

7. The guide device (10) according to claim 6, wherein the first opposing edges (21) include barriers formed on the outer surface (19) to abut the respective eyelids.

8. The guide device (10) according to any one of the preceding claims, wherein the inner surface (13) includes a medicament.

9. The guide device (10) according to claim 8, wherein the medicament is at least one of an antimicrobial agent, an anti-infection agent, and an anti-bacterial agent.

10. The guide device (10) according to any one of the preceding claims, wherein the base plate (11) is rotatable relative to the eye ball between at least a first orientation and a second orientation.
